# EUROPEAN PATENT SPECIFICATION

(11) **EP 0 942 660 B1**
(45) Date of publication and mention of the grant of the patent: **28.05.2003**
(21) Application number: 97913633.0
(22) Date of filing: 14.11.1997
(51) Int. Cl.: A23K 1/16, A23K 1/165, A23L 1/10, A23L 1/185

(54) **FOOD-INDUCED ANTISECRETORY PROTEINS**
DURCH LEBENSMITTEL INDUZIERTE ANTISEKRETORISCHE PROTEINE
PROTEINES ANTISECRETRICES INDUITE PAR L'ALIMENTATION

(30) Priority: 20.11.1996 SE 9604251
(43) Date of publication of application: 22.09.1999
(73) Proprietor: Rural Patent Svenska AB, 104 25 Stockholm (SE)
(72) Inventor: LANGE, Stefan, S-411 28 Göteborg (SE); GÖRANSSON, Leif, S-260 23 Kageröd (SE); LÖNNROTH, Ivar, S-431 69 Mölndal (SE)
(74) Representative: Perneborg, Henry T.
(86) International application number: SE9701918
(87) International publication number: WO98021978

(56) References cited:
- WO-A-91/09536
- US-A- 1 410 973
- US-A- 5 565 225
- J. VET. MED. B., Volume 40, 1993, L. GORANSSON et al., "Feed-Induced Lectins in Piglets", pages 478-484.

## Description

The present invention relates to the use of products having enzymatic activity for the preparation of a food inducing the formation of antisecretory proteins (ASP).

### Background of Invention

A heavy secretion of body fluids arises in a number of different situations. A large intake of food before a physical excertion readily results in secretion of body fluid into the intestine. The physical excertion as such results in a flux of fluid in muscles and joints. This phenomenon gives rise to stiffness and reduced performance. Irritation of the intestinal wall with different agents readily imparts an uncomfortably soft consistency to the motion.

In Sweden there are more than 10,000 ostomy-operated persons. About 1000 are added each year. A none too insignificant part of these persons is operated with small intestine ostomy (ileostomy). In cases where there is a secretion exceeding one litre daily, problems often arise owing to an abundant flux in the ostomy bag which requires frequent exchanges and emptying of the bag. For each individual, this brings about significant inconveniences with restrictions in social activities, working capacity, fluid deficiency and calorie and mineral deficiency. Persons having these problems have had difficulties in getting effective help.

During recent years important findings concerning the ability of the body to regulate the net flux of fluid and electrolytes in the intestine have been made. Various peptides having the capacity to enhance the resorption of fluid and electrolytes across the intestinal wall have been found. The most important of these hormone-like peptides are somatostatin and neuropeptide y (Krieger DT and Martin JB, *N. Engl. J. Med.* 304:876-885, 1981; Miller *J., Regulatory peptides* 4 (Suppl.): 203-208, 1985) as well as the so-called antisecretory proteins (Lange S. and Lönnroth I., *FEMS Microbiol. Letters* 24: 165-168, 1984; Lange S. and Lönnroth I., *Biochim. Biophys. Acta* 883: 138-144, 1986). ASP reverses the secretion and increases the resorption of fluid and electrolytes in the intestine.

### State of the Art

It is known from SE 9000028-2 (publication No. 466331) that the formation of an antisecretory factor (ASF) or antisecretory protein can be stimulated in animals by feeding the animals with a feed to which amino acids and/or sugars and/or amides in certain amounts have been added. Due to the formation of this antisecretory factor one can reduce diarrhoea of different causes in animals. By estimating the content of ASP by a method described in said patent, the amounts of amino acids and sugars can be adjusted so that an effective amount of ASP is formed at a cost which is commercially interesting.

It is also known from e.g. Khin-Maung-U and William Greenough III (*J. Pediatrics* 118, 72-85 (1991) that rice meal and wheat meal as well as decoctions thereof can be used for rehydratisation of diarrhoea. Salts are often added in order to compensate for the loss of these substances. The purpose of these preparations is only to compensate for the fluid loss already set in.

### Brief description of the Invention

The object of the invention is to provide a food alleviating or remedying the troubles and phenomena associated with the undesired secretion of body fluids described above. The object of the invention is attained by using products having enzymatic activity to provide a food which, when consumed, induces the formation of antisecretory proteins.

### Detailed description of the Invention

During the continued work of studying the formation of ASP, it has surprisingly been found that the formation of ASP is stimulated not only by the addition of amino acids and sugars but also by foods to which enzymes have been added which can hydrolyse the food's content of carbohydrates and proteins at such a rate that the amounts of sugars and amino acids known *per se* appear in the intestinal canal. It has then surprisingly been found that the formation of ASP can be controlled or governed by varying the amount and proportion of the product having enzymatic activity. Owing to this, foods can be prepared having a composition such that the content of ASP formed at repeated consumption can be predicted with a reasonable degree of safety. This is particularly significant since the dose response varies between different categories of individuals.

The discovery that malted cereals have the ability to provide effective amounts of sugars and amino acids is particularly surprising. This opens the possibility to prepare foods which are nourishing, palatable and have the ability to stimulate the formation of ASP.

The term "food" as used herein is intended to comprise food for human consumption as well as feed for animal consumption. The food is preferably a product in the form of bread, bisquits, pasta, grains and flakes, porridge and gruel but can also be a food preparation containing meat and meat products, fat and fat products or milk and milk products.

According to a preferred embodiment malted cereals are used in the preparation of the ASP-inducing food.

The term "cereals" as used herein is intended to comprise the usual kinds of cereals or grain such as wheat, barley, rye, oats, rice, corn, millet, durra and sorghum.

"Malted cereals" are healthy and fresh grain that has been subjected to malting. The malting means that the grain kernels are steeped and thereafter are allowed to germinate at a carefully controlled water content and temperature until its sprout germs have developed. The germination time is adjusted to the respective lot and variety. The germinated kernels are dried and desprouted. The drying can be driven so that the enzyme activity is changed to a more or less extent. The product then obtained is malt. The nutritive substances of the kernel has then, to a restricted extent, been hydrolysed and the enzymes of the sprout have been activated. This partial hydrolysis also facilitates the attack of the endogenous enzymes of the digestive system on the nutritive substances. It is obvious that a certain precooking or heat treatment also can increase the hydrolysis rate.

When preparing food products the malted cereals can be added in admixture with non-malted cereals in such proportions that ASP in induced when the food thus prepared is consumed.

It has been shown in tests that cereal products which also normally make up a considerable part of the daily food intake can be supplemented with enzymes or preferably malt products to obtain a food which, when consumed, provides the desired ASP induction.

The amounts and proportions of the malted and non-malted, if any, cereals required to provide the intended effect can easily be established by the skilled man by routine tests where the response to the induction of the food is measured according to the method stated in SE 9000028-2. Briefly, the method involves measuring a standardized secretion response in the small intestine of the rat.

It has been shown that the ASP level required in order to obtain the intended effect is at least 0.5 units per ml of blood.

It is obvious that foods prepared according to the invention can be varied in a great number of ways and be given different embodiments. Owing to this diet monotony can be avoided. The need of stimulation of different individuals to reach an effective ASP concentration can be met by measuring the response of food intake as stated. Through the invention one can also compensate for varying activity of enzyme preparations as well as for differences in enzymatic activity between malted cereals.

Further, it is obvious that the food can be formulated in a number of different ways in order also to meat the requirements of palatability and variation. Foods prepared on the basis of malted cereals can be prepared in the form of breakfast flakes, bread, rolls and pasta products, using known technique. When preparing products requiring moistening with water, e.g. when making bread, the recipes have to be changed based upon tha baker's known experiences. It is also obvious that the products can be formulated as a powder, intended to be stirred into water or lemonade or another fluid and consumed as a beverage.

As examples of meat products, in which the malted cereals can be contained, mention can be made of meat pudding containing groats or sausage pudding where the groats are added as malted product. The decisive thing is of cause that the food is formulated so that the desired stimulation of the formation of ASP is achieved.

The value of being able to prepare foods inducing ASP at a predetermined level is evident from the fact that there are many situations where a decreased secretion is desired, such as extreme body excertion. Thus, it is well known that athlets get problem with soft motion when pressing themselves to the extreme, simultanouously with intake of large food and liquid volumes in order to provide the body with energy-rich carbohydrates. Firemen and soldiers have similar problems and they also get soft motion owing to the stress situations they are subjected to. A special problem arises when driving fast airplanes; the pilots must, owing to the high G forces, wear a napkin which can be avoided if the motion is made more solid by a new diet. Foods prepared in accordance with the present invention have a great potential value in such situations.

The invention is further illustrated by means of the following non-limiting specific examples.

### Example 1

### Experiments with malted cereals to persons used for experimental purposes

A number of persons used for experimental purposes were allowed to try different breakfast meals consisting of different cereal products. Blood samples were taken before and after the trial period; from these blood samples antisecretory proteins (ASP) were isolated by means of affinity chromatography according to the method described in SE 9000028-2. The content of ASP in the samples was measured in a bioassay in rat according to a method previously described (Lange *S., FEMS Microbiol*. *Letters* 15: 239-242, 1982). Briefly, the method amounts to operating a ligated loop in the middle of the small intestine of the rat, the ASP sample is injected intravenously shortly before injecting cholera toxine, 3 µg, in the intestinal loop. After 5 hours the animal is sacrificed and the weight as well as the length of the freely dissected intestinal loop are measured; the response (mg fluid per cm intestine) of animals having received ASP sample is compared with that of control animals having received buffert only.

The diet given was:
1) bread baked with wheat-flour in mixture with 30% of "Frisk-plus" piglet feed (Göransson L. et al., *J. Vet. Med.*, B, 40: 478-484, 1993);
2) bread baked with wheat-flour in mixture with 30% of ordinary barley-flour;
3) same as 2) but with malted barley-flour;
4) flakes comprising malted oats.

The results of the experiments are stated in the table below wherein the initials of the persons subjected to the experiment are stated as well as the activity in units of ASP per ml (1 unit = the amount of ASP providing 50% inhibition of the cholera toxine response). The net amount of cereals added (not wheat-flour or other cereals taken by the persons subjected to the experiment after the meal comprising test cereals) is stated within brackets.

| **Day** | **Diet, days** | **Activity of ASP i blood, units/ml** | | |
|---|---|---|---|---|
| | | **EE** | **SL** | |
| -135 | - | 0,0 | 0,0 | |
| -150 | "Frisk+" bread, 8 d | 1,4 (15 g) | 0,9 (26 g) | |
| -52 | - | 0,0 | 0,0 | |
| -31 | barley bread ctr, 10 d | 0,0 (29 g) | 0,0 (50 g) | |
| 0 | - | 0,0 | 0,1 | |
| 8 | malted barley bread, 7 d | 1,0 (25 g) | 0,5 (50 g) | |
| 21 | - | 0,0 | 0,4 | |
| 28 | malted oat flakes, 13 d | 1,3 (25+25 g) | 0,6 (60 g) | |
| 37 | - | 0,6 | 1,1 | |
| 62 | - | 0,4 | 0,0 | |
| | | | | |

| | | **EJ** | **IJ** | **IL** |
|---|---|---|---|---|
| 0 | - | 0,0 | 0,0 | 0,1 |
| 12 | malted oat flakes, 10 d | 1,0 (25+25 g) | 0,7 (25+25 g) | 1,0 (25+25 g) |
| 19 | - | 0,5 | - | 0,8 |

Normally, ASP does not seein to appear in human blood. After intake of bread baked on " Frisk + " piglet feed, ASP was induced in the blood of EE and SL. These two persons then ate bread baked on ordinary barley-flour and malted barley-flour, respectively. The ordinary barley bread did not induce ASP. However, the malted barley bread induced ASP. Twelve days after EE and SL had stopped eating the bread, the ASP value had decreased to 0.0 in EE and 0.4 in SL. The same persons then ate malted oat flakes added to soured milk. Also in this case ASP was induced. Similar to the preceding experiment, the ASP value of EE increased to a high level during the trial period and then rapidly decreased whereas SL got the highest ASP value one week after the trial period. The experiment with malted oat flakes was repeated with three further persons. They all got high ASP values during the trial period; a certain increase was registered also the week after they had stopped eating the test flakes.

### Example 2

### Experiments with pig feed to which enzymes have been added

Experiments on pigs that just had been weaned were carried out in a way similar to what has previously been described by Göransson et al. (1993). A conventional piglet feed with no addition av antibiotics, closely similar to "Lantmännens Växfor", and the same feed digested with enzymes added (a mixture of α- and β-amylase) were given to 2 x 5 litters beginning three days before the weaning day. Blood samples were taken at the day of weaning (day 0) as well as six days after weaning (day 6). The result showed that no detectable amounts of ASP could be found in the blood of the control group whereas the test group had a level of 0.9 units/ml already at day 0 which level then increased to 1.5 units/ml (n = 10 per group).

### Example 3

During the experimental work it has been shown that rats have responses to antisecretorily inducing agents similar to that of humans. Consequently, for the skilled man it is simpler to carry out controlled experiments on rats than on humans. The method of measuring the induction of antisecretory effect in rat is described in SE 9000028-2.

In a traditional laboratory test, part of the rat feed was replaced by test material. The rats were fed before the experiment with control and test diets for seven days. On the eight day swelling was induced (secretion out) in the intestine by injection of 3.5 micrograms of cholera toxine. The weight of the swollen intestine was determined and its weight in relation to the intestine weight of the control group is a measure of the degree of antisecretory effect or inhibition of secretion.

In one experimental run the following intestine weights and inhibition degrees were registered:

| **Diet** | **Number of animals** | **Intestine weight, mg/cm** | **% inhibition** |
|---|---|---|---|
| Control feed | 3 | 453 ± 3 | - |
| 80% control feed | | | |
| and 20% steam | | | |
| treated oat grains | 3 | 443 ± 16 | 2 (not signif.) |
| 80% control feed | | | |
| and 20% malted wheat | 3 | 82 ± 5 | 82 (signif.) |

As is evident from the results above, a most significant inhibition of the secretion was achieved in the group of rats that received 20% of the feed as malted wheat or, expressed in another way, a significant degree of antisecretory effect was achieved.

## Claims

1. Use of products having enzymatic activity for the preparation of foodstuff inducing, when consumed, antisecretory proteins (ASP) regulating the flux of fluid and electrolytes in the intestine so that 1 ml of blood will contain at least 0.5 units of ASP.

2. The use according to claim 1, **characterized in that** the products having enzymatic activity are malted cereals.

3. The use according to claim 2, **characterized in that** the malted cereals are barley, wheat, rye or oats.

4. The use according to claim 2, **characterized in that** the malted cereals are rice, corn or sorghum.

5. The use according to any of claims 2-4, **characterized in that** the malted cereals are in admixture with non-malted ones.

6. The use according to claim 1, **characterized in that** the foodstuff prepared is breakfast flakes, bread, rolls or pasta products.

## Patentansprüche

1. Verwendung von Produkten mit enzymatischer Aktivität für die Herstellung von Nahrungsmitteln, die, wenn sie konsumiert werden, antisekretorische Proteine (ASP) induzieren, die den Strom von Flüssigkeiten und Elektrolyten im Darm regulieren, so daß 1 ml Blut mindestens 0,5 Units ASP enthalten wird.

2. Die Verwendung nach Anspruch 1, **dadurch gekennzeichnet, daß** die Produkte mit enzymatischer Aktivität gemälzte Getreide sind.

3. Die Verwendung nach Anspruch 2, **dadurch gekennzeichnet, daß** die gemälzten Getreide Gerste, Weizen, Roggen oder Hafer sind.

4. Die Verwendung nach Anspruch 2, **dadurch gekennzeichnet, daß** die gemälzten Getreide Reis, Mais oder Sorghum sind.

5. Die Verwendung nach einem der Ansprüche 2 bis 4, **dadurch gekennzeichnet, daß** die gemälzten Getreide in Beimischung zu nicht gemälzten Getreiden vorliegen.

6. Die Verwendung nach Anspruch 1, **dadurch gekennzeichnet, daß** die hergestellten Nahrungsmittel Frühstücksflocken, Brot, Brötchen oder Nudelprodukte sind.

## Revendications

1. Utilisation de produits ayant une activité enzymatique pour la préparation d'aliments induisant après consommation, des protéines antisécrétoires (ASP) régulant le flux de liquide et d'électrolytes dans l'intestin, de façon à ce que 1 ml de sang contienne au moins 0,5 unité d'ASP.

2. Utilisation selon la revendication 1, **caractérisée en ce que** les produits ayant une activité enzymatique sont des céréales maltées.

3. Utilisation selon la revendication 2, **caractérisée en ce que** les céréales maltées sont l'orge, le blé, le seigle ou l'avoine.

4. Utilisation selon la revendication 2, **caractérisée en ce que** les céréales maltées sont le riz, le maïs ou le sorgho.

5. Utilisation selon l'une quelconque des revendications 2 à 4, **caractérisée en ce que** les céréales maltées sont mélangées à d'autres céréales non maltées.

6. Utilisation selon la revendication 1, **caractérisée en ce que** l'aliment préparé est constitué de produits pour petit déjeuner, de type flocons, pains, rouleaux ou pâtes.
